# EUROPEAN PATENT SPECIFICATION

(11) **EP 3 519 029 B1**
(45) Date of publication and mention of the grant of the patent: **09.09.2020**
(21) Application number: 17772749.2
(22) Date of filing: 02.10.2017
(51) Int. Cl.: A61M 16/10, A61M 16/01, A61M 16/18, B01D 53/00

(54) **A SEDATION DEVICE**
VORRICHTUNG ZUR SEDIERUNG
DISPOSITIF DE SÉDATION

(30) Priority: 30.09.2016 EP 16191980
(43) Date of publication of application: 07.08.2019
(73) Proprietor: Sedana Medical Limited, Two Mile house Naas, County Kildare (IE)
(72) Inventor: OOMEN, Glen, Naas Co. Kildare (IE); FARRELL, Ron, Johnstown, Naas Conty Kildare (IE); CAREY, Pauric, Derrinturn, Carbury County Kildare (IE)
(74) Representative: Schütte, Gearoid
(86) International application number: PCT/EP2017/075014
(87) International publication number: WO 2018/060525

(56) References cited:
- WO-A1-00/02610
- WO-A1-00/21595
- WO-A1-97/14465
- WO-A1-99/33523
- WO-A1-2004/087244
- WO-A1-2004/098688
- WO-A1-2010/096299
- US-A- 5 337 739

## Description

### Introduction

This invention relates to a sedation device for volatile anaesthetic delivery to a patient.

### Background of the Invention

The invention particularly relates to a sedation device comprising a housing, an interior volume of the housing defining a housing deadspace volume, the housing having a ventilator chamber and an associated juxtaposed patient chamber communicating with the ventilator chamber, a filter mounted between the ventilator chamber and the patient chamber forming a common gas-permeable dividing wall between the ventilator chamber and the patient chamber, the ventilator chamber and the patient chamber communicating through the filter, the ventilator chamber having an inlet port for connection to a ventilator, the patient chamber having an outlet port for connection to a patient breathing tube, and an evaporator mounted within the patient chamber for delivery of a volatile sedative into the patient chamber.

The sedation device in use is mounted between a ventilator and a patient. Typically the ventilator sends fresh air to a patient through a tube or hose and exhaled air is exhausted back to the mechanical ventilator through a second hose. A Y-piece placed between the ventilator and patient separates the two hoses: the first hose carrying incoming fresh air flow from the ventilator; and the second hose, carrying exhaled CO₂ and moisture laden air to the ventilator. The sedation device is mounted between the Y-piece and the patient to deliver a volatile sedative into the air stream. To prevent aerosolized pathogens from being exhaled by the patient and contaminating the ventilator machine, a filter is incorporated in the sedation device mounted between the patient and the Y-piece. In addition to capturing particulate material and pathogens, filters of different media can also serve to retain the moisture exhaled by the patient and, during the inhalation phase of the cycle, return the moisture to the patient. Different absorbent or reflective media can be used in the filter to reflect additional exhalants, such as volatile anaesthetics or sedatives, back to the patient.

CO₂ rich air will always be exhaled into the ventilator circuit between the patient and the Y-piece. This volume of breathed air not involved in gas exchange is called the "deadspace". The larger the volume of this deadspace, the more exhaled CO₂ rich air may reside in it. How high the concentration of CO₂ becomes in a given volume of deadspace is dependent upon the tidal volume of the patient. For example, a patient with a deep, large tidal volume will flush much more of the CO₂ laden air from the deadspace with each breathing cycle than will a patient, such as a child, with a low tidal volume, taking small, shallow breaths. Unable to completely flush this space on the exhale, the smaller patient with a smaller tidal volume will quickly increase the concentration of the CO₂ in the breathing gas with each breathing cycle. It should be noted that the body does have a natural deadspace. This natural deadspace is the trachea or airway between lungs and mouth or nose, so there is always some natural rebreathing.

Increasing the concentration of CO₂ in the deadspace, however, can have severe consequences for the patient. The CO₂ concentration in the rebreathed or exhaled air can be considered in equilibrium with the CO₂ dissolved in the patient's blood. Therefore, in having too small a tidal volume or too great a deadspace and failing to adequately clear the CO₂ from it, the patient's blood CO₂ will rise accordingly, creating a state of respiratory acidosis in the patient. As the chemoreceptors and respiratory nuclei of the brain only respond to blood CO₂ concentrations, the normal physiological reflex to increasing blood CO₂ is increasing respiratory rate and depth to clear it. When this cannot happen or cannot happen naturally, as in a ventilated patient, the patient undergoes a very dangerous condition of respiratory stress. It should be noted that completely eliminating the deadspace is also undesirable. Decreasing the CO₂ concentration in the rebreathed air forces the respiratory nuclei of the brain to depress natural respiratory rates and tidal volume to maintain homeostasis.

In reducing deadspace, simply making the components of the circuit, such as the sedation device or an endotracheal tube, smaller is challenging. Doing so will typically increase the resistance of the air flowing through the components from inlet to outlet. This resistance creates a pressure gradient across the component, called pressure drop, making it particularly difficult for the patient to exhale quickly enough before receiving the next breath from the ventilator. The ventilation rate must be subsequently slowed to allow enough exhalation. There is risk that the compensating low ventilation rate will not permit sufficient oxygen to reach the patient.

Simply reducing the size of the sedation device reduces the size of the filter and this adversely affects the reflection rate of heat, moisture and volatile sedative. While the filter could be enlarged in the direction of air flow through the device to compensate for the reduction in filter cross-sectional area due to size reduction, this will increase the pressure drop across the filter which is undesirable for the patient.

From a manufacturing point of view producing separate sedation devices in a number of sizes, with different deadspace volumes suitable for different sized patients, for example adults of different lung capacity and children, is relatively expensive as different tooling, moulds, filters, etc. are required for each different size of sedation device.

The present invention is directed towards overcoming these problems.

WO 97/14465 discloses a sedation device for supplying a sedative to a patient with an absorption filter for absorption and desorption of sedative during use. The device includes a housing for mounting in an airflow path between a respirator and a patient. An evaporator located within the housing releases sedative into the airstream delivered through the device between the respirator and the patient. An absorption filter is mounted within the housing to absorb excess sedative exhaled by the patient and release the recovered sedative back into the airstream when the patient takes the next breath in order to conserve sdeative. The filter can be moved within the housing in use between a position in which the airstream fully passes through the filter and a position in which the airstream bypasses the filter. Another system for anaesthetic agent distribution is disclosed in WO 2004/087244.

### Summary of the Invention

According to the invention there is provided a sedation device for volatile anaesthetic delivery to a patient including a housing, an interior volume of the housing defining a housing deadspace volume, the housing having a ventilator chamber and an associated juxtaposed patient chamber communicating with the ventilator chamber, a filter mounted between the ventilator chamber and the patient chamber forming a common gas-permeable inner dividing wall between the ventilator chamber and the patient chamber, the ventilator chamber and the patient chamber communicating through the filter, the ventilator chamber having an inlet port for connection to a ventilator, the inlet port positioned at a side of the housing to promote air flow across a surface of the filter, the patient chamber having an outlet port for connection to a patient breathing tube, the outlet portion positioned at a side of the housing to promote air flow across a surface of the filter, an evaporator mounted within the patient chamber for delivery of a volatile sedative into the patient chamber, characterised in that at least one insert is fixedly mounted within the housing to reduce the deadspace volume within the housing, the or each insert being mounted against an outer wall of the ventilator chamber, or against an outer wall of the patient chamber spaced-apart from the filter to provide an air flow path therebetween. Advantageously as the device provides the ability to vary the internal deadspace volume of the housing of the sedation device during manufacture, it can be easily adapted for use with persons of different lung capacity, for example adults and children, for optimum treatment of the patient. Conveniently, from a manufacturing and a cost point of view, the device also makes it unnecessary to provide a range of housings in different sizes to accommodate different patients. Also, the insert could provide additional functions within the sedation device, such as delivery of drug, or the sensing of a drug or patient metabolite.

In another embodiment of the invention the insert nests with the outer wall of the ventilator chamber or the outer wall of the patient chamber, an outer face of the insert being shaped for complementary interengagement with an inner face of the associated outer wall.

In another embodiment of the invention the insert has an inner face shaped to facilitate air flow through the housing.

In another embodiment of the invention a multi-part insert is provided with said insert parts being fixedly mounted in one or both of the ventilator chamber and the patient chamber.

In another embodiment of the invention the insert has means to vary the volume of the insert.

In another embodiment of the invention the insert is expandable between a collapsed position and an expanded position.

In another embodiment of the invention the insert incorporates one or more sensors for sensing one or more parameters of air delivered through the housing.

In another embodiment of the invention a first insert is provided for mounting in the ventilator chamber and a second insert is provided for mounting in the patient chamber.

In another embodiment of the invention the insert comprises a drug-eluting insert.

In another embodiment of the invention the insert is nestable with an inner face of the outer wall of the ventilator chamber and a central air distribution fin is mounted on an inner face of the insert projecting outwardly therefrom in alignment with the inlet port.

In another embodiment of the invention the insert is adapted to provide a colour change indication when exposed over time to exhaled pharmacological, metabolic or pathogenic agents to indicate the presence of a pathogen, or an undesirable concentration of a chemical. The colour change indication may be due to a time related change of colour to indicate the device has been in use for a preset period and is depleted.

In another embodiment of the invention the insert incorporates a nebulizer, or a nebulizer is mounted thereon.

In another embodiment of the invention the insert incorporates a humidifier, or a humidifier is mounted thereon.

In another embodiment of the invention the insert is for mounting in the patient chamber and has a central slot aligned with the outlet port for reception of the evaporator mounted in the patient chamber.

In another embodiment of the invention the insert incorporates means for mounting an evaporator thereon.

In another embodiment of the invention the insert is for mounting the patient chamber and the insert incorporates an evaporator.

In another embodiment of the invention the insert is for mounting in the patient chamber and comprises a plurality of vanes projecting inwardly from an inner face of the insert.

In another embodiment of the invention the vanes are porous to facilitate delivery of a volatile material through the vanes into the patient chamber.

Also disclosed is a method for manufacturing a sedation device of the invention wherein the method includes the step of mounting and fixing at least one insert within the housing to reduce the internal deadspace volume of the housing to a selected desirable internal deadspace volume.

### Brief Description of the Drawings

The invention will be more clearly understood by the following description of some embodiments thereof, given by way of example only with reference to the accompanying drawings, in which:
Fig. 1 is a sectional perspective view of a sedation device according to the invention;
Fig. 2 is a view similar to Fig. 1 showing a housing portion of the device;
Fig. 3 is a perspective view of an insert portion of the device;
Fig. 4 is a perspective view of another insert portion of the device;
Fig. 5 is a schematic illustration of a sedation device in use;
Fig. 6 is a sectional perspective view of another sedation device according to the invention;
Fig. 7 is a perspective view of an insert forming portion of the device of Fig. 6;
Fig. 8 is a perspective view showing an underside of the insert of Fig. 7.
Fig. 9 is a view similar to Fig. 8 showing the insert in another condition of use;
Figs. 10-17 are various views of other insert designs according to the invention; and
Fig. 18 is a schematic sectional elevational view of another sedation device of the invention.

### Detailed Description of the Preferred Embodiments

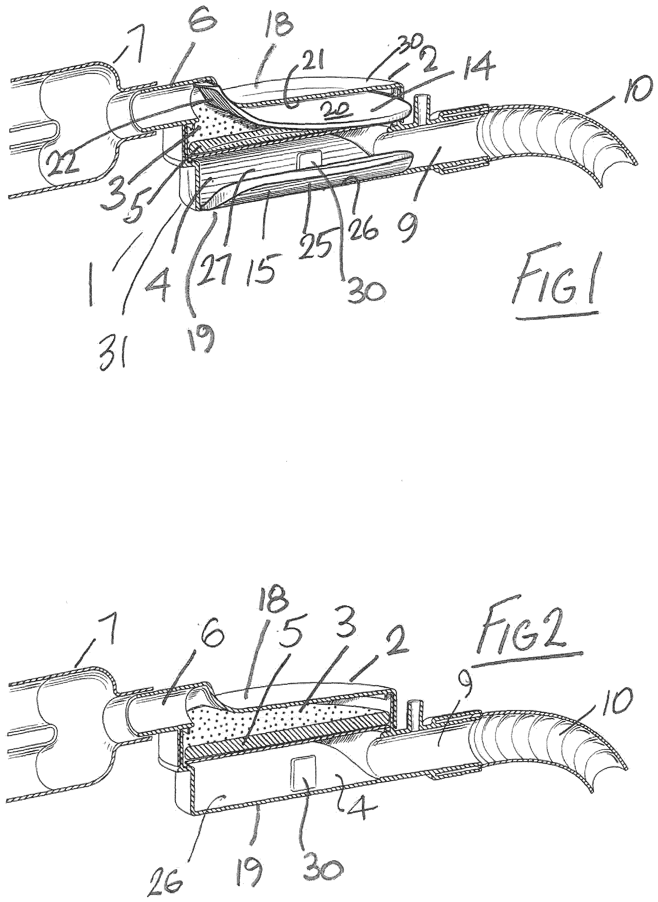

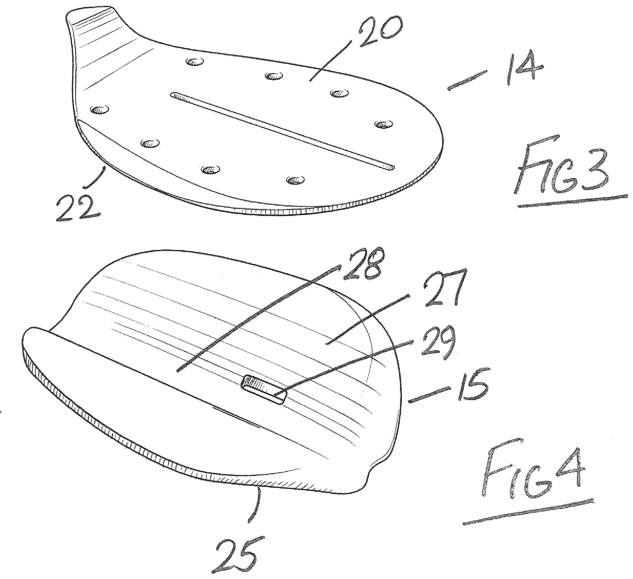

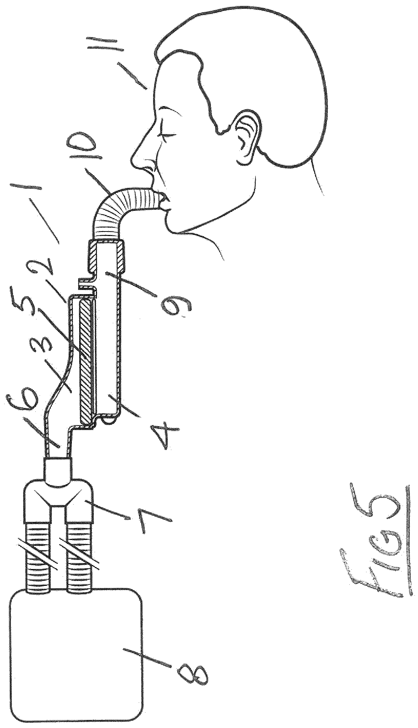

Referring to the drawings, and initially Figs. 1 to 5 thereof, there is illustrated a sedation device according to the invention indicated generally by the reference numeral 1. The sedation device 1 comprises a housing 2 having a ventilator chamber 3 and an associated patient chamber 4 in communication with the ventilator chamber 3. A filter 5 is mounted between the ventilator chamber 3 and the patient chamber 4 and forms a common gas-permeable inner dividing wall between the ventilator chamber 3 and the patient chamber 4. An inlet port 6 is provided on the ventilator chamber 3 for connection via a Y-piece 7 to a ventilator 8 (Fig. 5). An outlet port 9 of the patient chamber 4 connects via a patient breathing tube 10 with a patient 11 (Fig. 5). An evaporator (not shown) is mounted within the patient chamber 4 for delivery of a volatile sedative into the patient chamber 4 in use. An interior volume of the housing 2 defines a housing deadspace volume, which might for example be about 110 ml or 120 ml. In accordance with the present invention insert means is provided for varying the internal deadspace volume of the housing 2, said insert means in this case comprising an associated pair of inserts, namely a first insert 14 which is fixedly mounted in the ventilator chamber 3 and a second insert 15 which is fixedly mounted in the patient chamber 4. One or both of these inserts 14, 15 can be fixedly mounted within the housing 2 during manufacture to vary the internal deadspace volume of the housing 2 as required to suit different patients.

It will be noted that each of the inserts 14, 15 are nestably engagable with an inner face of an outer wall of the housing 2, spaced-apart from the filter 5 to provide an air flow path therebetween. An outer face 20 of the first insert 14 nestably engages against an inner face 21 at a top 18 of the outer wall of the housing 2. An inner face 22 of the first insert 14 is shaped to facilitate smooth air flow through the ventilator chamber 3 of the housing 2 across a surface of the filter 5.

Similarly, an outer face 25 of the second insert 15 is shaped for nesting engagement against an inner face 26 of a bottom 19 of the outer wall of the housing 2 within the patient chamber 4. An inner face 27 of the second insert 15 is shaped to promote smooth air flow through the patient chamber 4 of the housing 2. A central channel 28 is provided in the second insert 15 to facilitate mounting means for evaporating or nebulizing a drug within the patient chamber 4. Through-holes 29 in the insert 15 allow through passage of filter support posts 30 which project inwardly from the bottom 19 of the outer wall.

The housing 2 is provided in two interlocking parts, namely an upper part 30 forming the ventilator chamber 3 and an associated lower part 31 forming the patient chamber 4 for complementary inter-engagement to form the housing 2. These parts 30, 31 snap together and may be glued, welded or otherwise fixed together to form the housing 2.

The filter 5 comprises an absorbent carbon felt filter adjacent to an anti-microbial and anti-viral filter. The activated carbon on the filter 5 functions to reflect heat, moisture and volatile anaesthetic back to the patient. The anti-bacterial and anti-viral filter serves to protect the ventilator circuit from pathogenic contamination.

In use, the sedation device 1 can be used either with one or both of the inserts 14, 15 (as shown in Fig. 1) to provide a sedation device 1 with different internal deadspace volumes suited to different patients. For example, without the inserts 14, 15 the internal deadspace volume of the housing 2 might be about 100ml or 110ml, whereas by use of a suitable insert 14, 15 the internal deadspace volume of the housing 2 might be readily easily reduced to 30ml, 50ml or 60 ml for example, or indeed any desired internal deadspace volume in the range 30ml to 110ml. During manufacture of the sedation device 1 suitable inserts are selected and fixed within the housing 2 so that upon assembly of the housing 2 a desired internal housing deadspace volume is achieved. Thus conveniently a single housing 2 of maximum required deadspace volume can be produced and by securing inserts of different size within the housing 2, sedation devices 1 having a range of internal deadspace volumes can be produced.

While the inserts 14, 15 allow adjustment of the internal deadspace volume of the housing 2, at the same time they are designed to minimise flow resistance and pressure drop across the sedation device 1 when they are in use to ensure a smooth flow of air through the device 1 for patient safety and comfort. It will be noted also that the internal deadspace volume can be reduced without reducing the filter size which thus maintains a good reflection capability.

The inserts 14, 15 can be made of a material that is absorbent and reflective to augment the capacity of the filter 5 to return moisture or volatile pharmacological agents to the patient. Further, the insert 14, 15 could be used to deliver a drug to the patient through passive evaporation, active vaporisation, nebulisation, atomisation or be made of or coated or impregnated with a drug eluting material. It is also envisaged that the insert could be adapted to deliver two or more drugs.

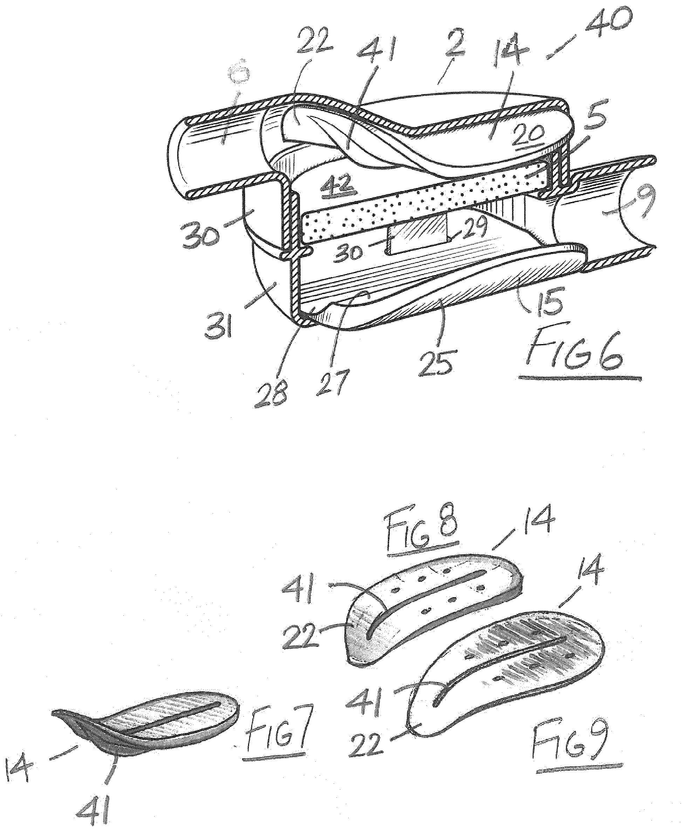

Referring now to Figs. 6 to 9 there is shown another sedation device according to the invention indicated generally by the reference numeral 40. Parts similar to those described previously are assigned the same reference numerals. In this case the first insert 14 is adapted to provide a change of colour indication (as shown in Fig. 9) when exposed over time to exhaled pharmacological, metabolic or pathogenic agents to indicate the presence of a pathogen, undesirable concentration of a chemical or that the device is worn and needs to be replaced. In this case also a central air distribution fin 41 is mounted on the inner face 22 of the insert 14, projecting outwardly therefrom in alignment with the inlet port 6, that is in alignment with a centre of the inlet port 6. This facilitates an even distribution of air across the upper surface 42 of the filter 5.

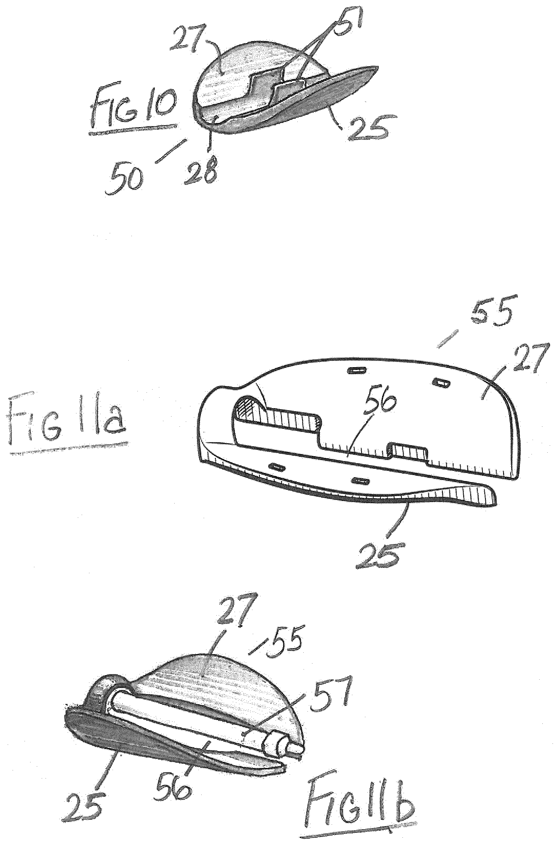

Figs. 10 to 17 show various other insert designs.

Fig. 10 shows another insert 50 for mounting in the patient chamber 4. Upstanding supports 51 are provided at opposite sides of the channel 28 to support the filter 5 spaced-apart from the inner face 27 to provide an air flow path between the filter 5 and the insert 50.

In Fig. 11 an insert 55 has a central slot 56 to accommodate an evaporator 57 when the insert 55 is mounted in the patient chamber 4. Fig. 11a and 11b show two design options to achieve this.

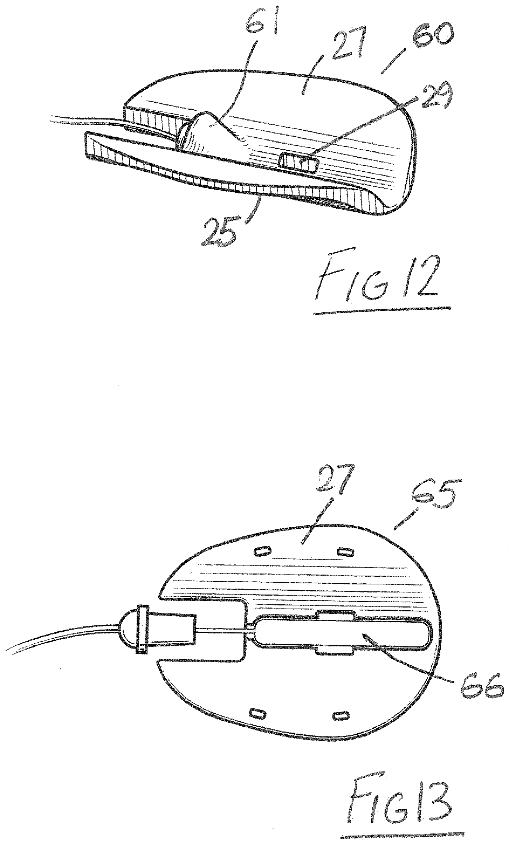

Another insert 60 is shown in Fig. 12 which in this case incorporates an evaporator. A connector 61 can be connected to an associated reservoir, of anaesthetic for example or a volatile medication, for delivery into the insert 60 and evaporation through the inner face 27 of the insert 60 into the patient chamber 4 of the device 1.

Fig. 13 shows an insert 65 which incorporates a sensor 66 to sense desired parameters such as pressure or humidity or possibly the presence of a particular chemical. Instead of the sensor 66 a vaporiser, nebulizer or humidifier could be mounted on the insert 65.

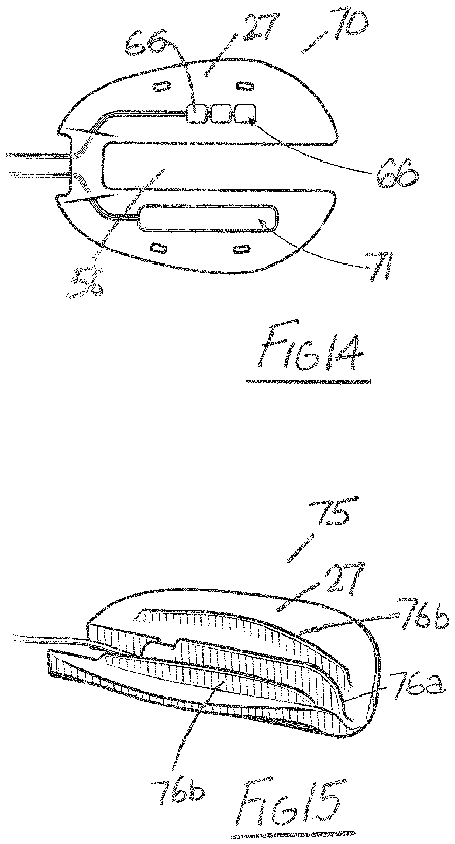

Fig 14 shows an insert 70 similar to that shown in Fig. 11 but in this case incorporating a number of sensors 66 and a separate delivery device 71 for delivery of a volatile medication into the patient chamber 4 of the device 1.

In Fig. 15 there is shown an insert 75 incorporating a number of upstanding vanes 76 which project outwardly of the inner face 27 of the insert 75 to increase the surface area for emission of volatile material into the patient chamber 4 and control the flow of air through the patient chamber 4. The vanes 76 may be substantially parallel to the outlet port 9 when the insert 75 is mounted in the patient chamber 4. That is a central vane 76a is centrally aligned with the outlet port 9 and outer vanes 76b are substantially parallel to the central vane 76a or curve inwardly towards the central vane 76a in the direction of the outlet port 9.

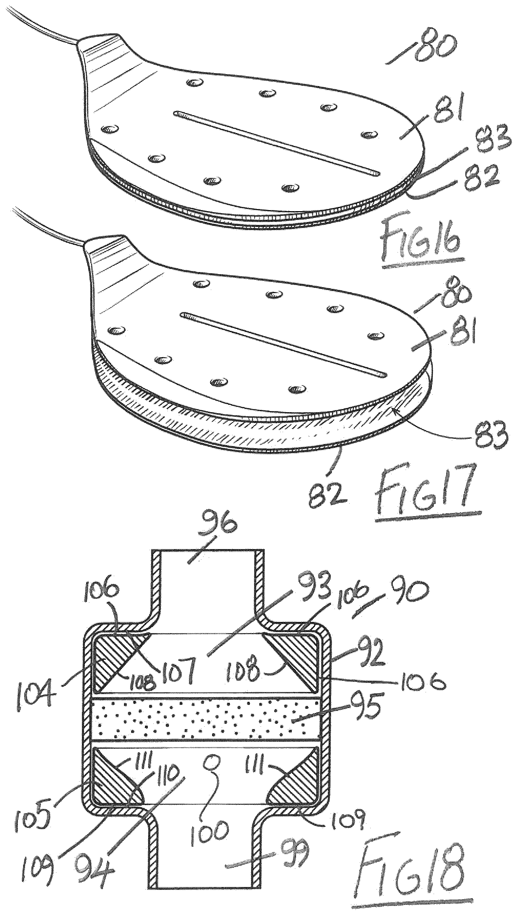

A variable volume insert 80 is shown in Fig. 16 and Fig. 17. In this case the insert 80 has upper and lower panels 81, 82 separated by an inflatable bladder or bag 83 mounted between the panels 81, 82. Inflating or deflating the bag 83 will vary the volume of the insert 80. Any suitable fluid may be used to inflate the bag 83, air generally being the most convenient.

Referring to Fig. 18 there is illustrated another sedation device according to the invention indicated generally by the reference numeral 90. The sedation device 90 comprises a generally cylindrical housing 92 having a ventilator chamber 93 and an associated patient chamber 94 in communication with the ventilator chamber 93. A filter 95 is mounted between the ventilator chamber 93 and the patient chamber 94 and forms a common gas-permeable dividing wall between the ventilator chamber 93 and the patient chamber 94. An inlet port 96 is provided on the ventilator chamber 93 for connection to the ventilator 8. An outlet port 99 of the patient chamber 94 connects via the patient breathing tube 10 with a patient 11. An evaporator 100 is mounted within the patient chamber 94 to deliver a volatile sedative into the patient chamber 94 in use. Air flows straight through the housing 92 between the inlet port 96, and the outlet port 99 through the filter 95. The means for varying the internal volume of the housing 92, in this case comprises an associated pair of inserts, namely a first insert 104 for mounting in the ventilator chamber 93 and a second insert 105 for mounting in the patient chamber 94. One or both of these inserts can be mounted and fixed within the housing 92 to vary the internal volume of the housing 92 as required to suit different patients. It will be noted that each of the inserts 104, 105 are nestably engagable with an inner wall of the housing 92. An outer face 106 of the first insert 104 nestably engages against an inner face 107 of the housing wall at a top of the housing 92. An inner face 108 of the first insert 104 is shaped to facilitate smooth air flow through the ventilator chamber 93 of the housing 92. In this case the inner face 108 is tapered outwardly from the inlet port 96 to the filter 95. Similarly, an outer face 109 of the second insert 105 is shaped for nesting engagement against an inner face 110 of the housing wall at a bottom of the housing 92 within the patient chamber 94. An inner face 111 of the second insert 105 is shaped to promote smooth air flow through the patient chamber 94 of the housing 92. The inner face 111 tapers outwardly from the outlet port 99 to the filter 95.

Advantageously the invention facilitates fine tuning of the deadspace volume of the sedation device to accommodate different patient requirements without needing to manufacture sedation devices in a myriad of individual volumes that might be clinically useful. The invention being proposed is a sedation device and an associated insert, or series of inserts that can be inserted into the sedation device by the manufacturer to reduce the internal volume and deadspace of that device. The invention firstly increases the range of tidal volumes over which an existing sedation device could be clinically useful without needing to manufacture a costly array of entirely unique housings and filters. Secondly, such an insert would also carefully consider how the air flows over it and through the device, and is designed to reduce resistance to keep pressure drop to a minimum.

It would also be advantageous and add value, with such an insert proposed by the invention, to increase the functionality of an existing sedation device. Additional functions could be designed into the insert to include: a means of delivering additional agents, such as volatile or aerosolized drugs; a chemical or physical sensory capability; an ability to indicate wear or use; or an ability to dynamically alter the internal volume of the sedation device.

In this specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described which may be varied in construction and detail within the scope of the appended claims.

## Claims

1. A sedation device (1) for volatile anaesthetic delivery to a patient, including:
a housing (2);
an interior volume of the housing (2) defining a housing deadspace volume;
the housing (2) having a ventilator chamber (3) and an associated juxtaposed patient chamber (4) communicating with the ventilator chamber(3);
a filter (5) mounted between the ventilator chamber (3) and the patient chamber (4) forming a common gas-permeable dividing wall between the ventilator chamber (3) and the patient chamber (4), the ventilator chamber (3) and the patient chamber (4) communicating through the filter (5);
the ventilator chamber (3) having an inlet port (6) for connection to a ventilator (8), the inlet port (6) positioned at a side of the housing (2) to promote air flow across a surface of the filter (5);
the patient chamber (4) having an outlet port (9) for connection to a patient breathing tube (10), the outlet port (9) positioned at a side of the housing (2) to promote air flow across a surface of the filter (5);
an evaporator mounted within the patient chamber (4) for delivery of a volatile sedative into 1 the patient chamber (4);
**characterised in that** at least one insert (14, 15) is fixedly mounted within the housing (2) to reduce the deadspace volume within the housing (2), the or each insert (14, 15) being mounted against an outer wall of the ventilator chamber (3) or against an outer wall of the patient chamber (4) spaced-apart from the filter (5) to provide an air flow path therebetween.

2. The sedation device (1) as claimed in claim 1 wherein the insert (14, 15) nests with the outer wall of the ventilator chamber (3) or the outer wall of the patient chamber (4), an outer face of the insert (14, 15) being shaped for complementary interengagement with an inner face of the associated outer wall.

3. The sedation device (1) as claimed in claim 2 wherein the insert (14, 15) has an inner face shaped to facilitate air flow through the housing (2).

4. The sedation device (1) as claimed in any preceding claim wherein a multi-part insert (14, 15) is provided with said insert parts (14, 15) being fixedly mounted in one or both of the ventilator chamber (3) and the patient chamber (4).

5. The sedation device (1) as claimed in any preceding claim wherein the insert (80) has means to vary the volume of the insert (80).

6. The sedation device (1) as claimed in claim 5 wherein the insert (80) is expandable between a collapsed position and an expanded position.

7. The sedation device (1) as claimed in any preceding claim wherein the insert (65) incorporates one or more sensors (66) for sensing one or more parameters of air delivered through the housing (2).

8. The sedation device (1) as claimed in any preceding claim wherein a first insert (14) is provided for mounting in the ventilator chamber (3) and a second insert (15) is provided for mounting in the patient chamber (4).

9. The sedation device (1) as claimed in any preceding claim wherein the insert comprises a drug-eluting insert.

10. The sedation device (1) as claimed in any preceding claim, wherein the insert (14) is nestable with an inner face of the outer wall of the ventilator chamber (3) and a central air distribution fin (41) is mounted on an inner face of the insert (14) projecting outwardly therefrom in alignment with the inlet port (6).

11. The sedation device (1) as claimed in any preceding claim, wherein the insert (14) is adapted to provide a colour change indication when exposed over time to exhaled pharmacological, metabolic or pathogenic agents to indicate the presence of a pathogen, or an undesirable concentration of a chemical.

12. The sedation device (1) as claimed in any preceding claim wherein the insert incorporates a nebulizer.

13. The sedation device (1) as claimed in any preceding claim wherein the insert incorporates a humidifier.

14. The sedation device (1) as claimed in any preceding claim wherein the insert (15) is for mounting in the patient chamber (4) and the insert (15) has a central slot (28) aligned with the outlet port (9) for reception of the evaporator mounted in the patient chamber (4).

15. The sedation device (1) as claimed in any preceding claim wherein the insert incorporates means for mounting an evaporator thereon.

## Patentansprüche

1. Sedierungsvorrichtung (1) zum Zuführen von volatilem Anästhetika zu einem Patienten, umfassend:
ein Gehäuse (2);
einen inneres Volumen des Gehäuses (2), das ein Gehäusetotraumvolumen definiert;
wobei das Gehäuse (2) eine Beatmungsgerätkammer (3) und eine assoziierte danebenliegende Patientenkammer (4), die mit der Beatmungsgerätkammer (3) in Verbindung steht, aufweist;
einen Filter (5), der zwischen der Beatmungsgerätkammer (3) und der Patientenkammer (4) angebracht ist und eine gemeinsame gasdurchlässige Trennwand zwischen der Beatmungsgerätkammer (3) und der Patientenkammer (4) bildet, wobei die Beatmungsgerätkammer (3) und die Patientenkammer (4) durch den Filter (5) miteinander in Verbindung stehen;
wobei die Beatmungsgerätkammer (3) einen Einlassstutzen (6) zur Verbindung mit einem Beatmungsgerät (8) aufweist, wobei der Einlassstutzen (6) auf einer Seite des Gehäuses (2) positioniert ist, um den Luftstrom über eine Oberfläche des Filters (5) zu begünstigen;
wobei die Patientenkammer (4) einen Auslassstutzen (9) zur Verbindung mit einem Patientenatemtubus (10) aufweist, wobei der Auslassstutzen (9) auf einer Seite des Gehäuses (2) positioniert ist, um den Luftstrom über eine Oberfläche des Filters (5) zu begünstigen;
einen in der Patientenkammer (4) angebrachten Verdunster zur Zufuhr eines volatilen Sedativs in die Patientenkammer (4);
**dadurch gekennzeichnet, dass** mindestens ein Einsatz (14, 15) fest in dem Gehäuse (2) angebracht ist, um das Totraumvolumen in dem Gehäuse (2) zu verringern, wobei der oder jeder Einsatz (14, 15) an einer Außenwand der Beatmungsgerätkammer (3) oder an einer Außenwand der Patientenkammer (4) von dem Filter (5) beabstandet angebracht ist, um einen Luftströmungsweg dazwischen bereitzustellen.

2. Sedierungsvorrichtung (1) nach Anspruch 1, wobei der Einsatz (14, 15) mit der Außenwand der Beatmungsgerätkammer (3) oder der Außenwand der Patientenkammer (4) verschachtelt ist, wobei eine Außenoberfläche des Einsatzes (14, 15) für den komplementären gegenseitigen Eingriff mit einer Innenoberfläche der assoziierten Außenwand geformt ist.

3. Sedierungsvorrichtung (1) nach Anspruch 2, wobei der Einsatz (14, 15) eine Innenoberfläche aufweist, die geformt ist, um den Luftstrom durch das Gehäuse (2) zu erleichtern.

4. Sedierungsvorrichtung (1) nach einem der vorangehenden Ansprüche, wobei ein mehrteiliger Einsatz (14, 15) bereitgestellt ist, sodass die Einsatzteile (14, 15) fest in der Beatmungsgerätkammer (3) und/oder der Patientenkammer (4) angebracht sind.

5. Sedierungsvorrichtung (1) nach einem der vorangehenden Ansprüche, wobei der Einsatz (80) Mittel zum Verändern des Volumens des Einsatzes (80) aufweist.

6. Sedierungsvorrichtung (1) nach Anspruch 5, wobei der Einsatz (80) zwischen einer eingefallenen Stellung und einer expandierten Stellung expandierbar ist.

7. Sedierungsvorrichtung (1) nach einem der vorangehenden Ansprüche, wobei der Einsatz (65) einen oder mehrere Sensoren (66) zum Abfühlen von einem oder mehreren Parametern von durch das Gehäuse (2) zugeführter Luft einbezieht.

8. Sedierungsvorrichtung (1) nach einem der vorangehenden Ansprüche, wobei ein erster Einsatz (14) zum Anbringen in der Beatmungsgerätkammer (3) bereitgestellt ist und ein zweiter Einsatz (15) zum Anbringen in der Patientenkammer (4) bereitgestellt ist.

9. Sedierungsvorrichtung (1) nach einem der vorangehenden Ansprüche, wobei der Einsatz einen medikamentenbeschichteten Einsatz umfasst.

10. Sedierungsvorrichtung (1) nach einem der vorangehenden Ansprüche, wobei der Einsatz (14) mit einer Innenoberfläche der Außenwand der Beatmungsgerätkammer (3) verschachtelbar ist und eine zentrale Luftverteilungslamelle (41) auf einer Innenoberfläche des Einsatzes (14) angebracht ist, die auf den Einlassstutzen (6) ausgerichtet davon nach außen vorsteht.

11. Sedierungsvorrichtung (1) nach einem der vorangehenden Ansprüche, wobei der Einsatz (14) dazu angepasst ist, eine Farbänderungsanzeige bereitzustellen, wenn er mit der Zeit ausgeatmeten pharmakologischen, metabolischen oder pathogenen Wirkstoffen ausgesetzt ist, um die Anwesenheit eines Pathogens oder einer unerwünschten Konzentration einer Chemikalie anzuzeigen.

12. Sedierungsvorrichtung (1) nach einem der vorangehenden Ansprüche, wobei der Einsatz einen Vernebler einbezieht.

13. Sedierungsvorrichtung (1) nach einem der vorangehenden Ansprüche, wobei der Einsatz einen Befeuchter einbezieht.

14. Sedierungsvorrichtung (1) nach einem der vorangehenden Ansprüche, wobei der Einsatz (15) zum Anbringen in der Patientenkammer (4) bestimmt ist und der Einsatz (15) einen auf den Auslassstutzen (9) ausgerichteten zentralen Schlitz (28) zum Aufnehmen des in der Patientenkammer (4) angebrachten Verdunsters aufweist.

15. Sedierungsvorrichtung (1) nach einem der vorangehenden Ansprüche, wobei der Einsatz Mittel zum Anbringen eines Verdunsters daran einbezieht.

## Revendications

1. Dispositif de sédation (1) pour la délivrance d'un anesthésique volatil à un patient, incluant :
un boîtier (2) ;
un volume intérieur du boîtier (2) définissant un volume inutilisé de boîtier ;
le boîtier (2) ayant une chambre de ventilateur (3) et une chambre de patient juxtaposée associée (4) communiquant avec la chambre de ventilateur (3) ;
un filtre (5) monté entre la chambre de ventilateur (3) et la chambre de patient (4) formant une paroi de séparation commune perméable au gaz entre la chambre de ventilateur (3) et la chambre de patient (4), la chambre de ventilateur (3) et la chambre de patient (4) communiquant par le filtre (5) ;
la chambre de ventilateur (3) ayant un orifice d'entrée (6) pour la connexion à un ventilateur (8), l'orifice d'entrée (6) positionné au niveau d'un côté du boîtier (2) pour favoriser l'écoulement d'air à travers une surface du filtre (5) ;
la chambre de patient (4) ayant un orifice de sortie (9) pour la connexion à un tube de respiration de patient (10), l'orifice de sortie (9) positionné au niveau d'un côté du boîtier (2) pour favoriser l'écoulement d'air à travers une surface du filtre (5) ;
un évaporateur monté au sein de la chambre de patient (4) pour la délivrance d'un sédatif volatil jusque dans la chambre de patient (4) ;
**caractérisé en ce qu'**au moins une pièce rapportée (14, 15) est montée fixement au sein du boîtier (2) pour réduire le volume d'espace inoccupé au sein du boîtier (2), la ou chaque pièce rapportée (14, 15) étant montée contre une paroi externe de la chambre de ventilateur (3) ou contre une paroi externe de la chambre de patient (4) espacée du filtre (5) pour fournir un trajet d'écoulement d'air entre celles-ci.

2. Dispositif de sédation (1) selon la revendication 1 dans lequel la pièce rapportée (14, 15) s'imbrique avec la paroi externe de la chambre de ventilateur (3) ou la paroi externe de la chambre de patient (4), une face externe de la pièce rapportée (14, 15) étant façonnée pour un emboîtement complémentaire avec une face interne de la paroi externe associée.

3. Dispositif de sédation (1) selon la revendication 2 dans lequel la pièce rapportée (14, 15) a une face interne façonnée pour faciliter l'écoulement d'air à travers le boîtier (2).

4. Dispositif de sédation (1) selon l'une quelconque des revendications précédentes dans lequel une pièce rapportée en plusieurs parties (14, 15) est fournie avec lesdites parties de pièce rapportée (14, 15) étant montées fixement dans l'une de la chambre de ventilateur (3) et de la chambre de patient (4) ou dans les deux.

5. Dispositif de sédation (1) selon l'une quelconque des revendications précédentes dans lequel la pièce rapportée (80) dispose d'un moyen pour varier le volume de la pièce rapportée (80).

6. Dispositif de sédation (1) selon la revendication 5 dans lequel la pièce rapportée (80) est capable de déploiement entre une position repliée et une position déployée.

7. Dispositif de sédation (1) selon l'une quelconque des revendications précédentes dans lequel la pièce rapportée (65) comporte un ou plusieurs détecteurs (66) destinés à détecter un ou plusieurs paramètres de l'air délivré par le boîtier (2).

8. Dispositif de sédation (1) selon l'une quelconque des revendications précédentes dans lequel une première pièce rapportée (14) est destinée à être montée dans la chambre de ventilateur (3) et une deuxième pièce rapportée (15) est destinée à être montée dans la chambre de patient (4).

9. Dispositif de sédation (1) selon l'une quelconque des revendications précédentes dans lequel la pièce rapportée comprend une pièce rapportée éluant un médicament.

10. Dispositif de sédation (1) selon l'une quelconque des revendications précédentes, dans lequel la pièce rapportée (14) est capable d'être imbriquée avec une face interne de la paroi externe de la chambre de ventilateur (3) et une ailette centrale de distribution d'air (41) est montée sur une face interne de la pièce rapportée (14) faisant saillie vers l'extérieur de celle-ci dans l'alignement de l'orifice d'entrée (6).

11. Dispositif de sédation (1) selon l'une quelconque des revendications précédentes, dans lequel la pièce rapportée (14) est conçue pour fournir une indication de changement de couleur quand elle est exposée dans le temps à des agents pharmacologiques, métaboliques ou pathogènes exhalés pour indiquer la présence d'un pathogène, ou d'une concentration indésirable d'un produit chimique.

12. Dispositif de sédation (1) selon l'une quelconque des revendications précédentes dans lequel la pièce rapportée comporte un nébuliseur.

13. Dispositif de sédation (1) selon l'une quelconque des revendications précédentes dans lequel la pièce rapportée comporte un humidificateur.

14. Dispositif de sédation (1) selon l'une quelconque des revendications précédentes dans lequel la pièce rapportée (15) est destinée à être montée dans la chambre de patient (4) et la pièce rapportée (15) a une fente centrale (28) alignée avec l'orifice de sortie (9) pour la réception de l'évaporateur monté dans la chambre de patient (4).

15. Dispositif de sédation (1) selon l'une quelconque des revendications précédentes dans lequel la pièce rapportée comporte un moyen destiné à monter un évaporateur sur celle-ci.
